# EUROPEAN PATENT SPECIFICATION

(11) **EP 2 603 219 B1**
(45) Date of publication and mention of the grant of the patent: **17.09.2014**
(21) Application number: 11739097.1
(22) Date of filing: 05.08.2011
(51) Int. Cl.: A61K 31/495, A61K 31/497, A61P 25/18

(54) **COMBINATION OF GLYT1 COMPOUND WITH ANTIPSYCHOTICS**
KOMBINATION AUS EINER GLYT1-VERBINDUNG MIT ANTIPSYCHOTIKA
COMBINAISON D'UN COMPOSÉ GLYT1 AVEC DES AGENTS ANTIPSYCHOTIQUES

(30) Priority: 09.08.2010 EP 10172316
(43) Date of publication of application: 19.06.2013
(73) Proprietor: F.Hoffmann-La Roche AG, 4070 Basel (CH)
(72) Inventor: ALBERATI, Daniela, CH-4058 Basel (CH); MOREAU, Jean-Luc, F-68460 Lutterbach (FR); WETTSTEIN, Joseph G., CH-4051 Basel (CH)
(74) Representative: Poppe, Regina
(86) International application number: PCT/EP2011/063533
(87) International publication number: WO 2012/019970

(56) References cited:
- WO-A1-2006/106425
- PINARD ET AL: "Selective GlyT1 Inhibitors: Discovery of [4-(3-Fluoro-5-trifluorometh ylpyridin-2-yl)piperazin-1-yl]-[5-methanes ulfonyl-2-((S)-2,2,2-triflu oro-1-methylethoxy)phenyl]methanone (RG1678), a Promising Novel Medicine To Treat Schizophrenia", JOURNAL OF MEDICINAL CHEMISTRY, vol. 53, 21 May 2010 (2010-05-21), pages 4603-4614, XP000002658697,

## Description

The present invention relates to a pharmaceutical combination of a glycine transporter inhibitor (GlyT1) and an atypical antipsychotic drug which may be used for the treatment of positive and negative symptoms of schizophrenia.
Schizophrenia is a severe and chronic mental illness, with prevalence estimates ranging from 1.4 to 4.6 per 1000 population [2.1]. Schizophrenic disorders are caused by a combination of genetic and environmental factors, which include probable neurodevelopmental abnormalities in gray and white matter structures. Underlying the symptomatic phenomena, disturbances in monoaminergic and glutamatergic neurotransmission (e.g. dopamine, serotonin, adrenaline, noradrenaline, glutamate) have been proposed.

These pathways are widely present in the CNS and, thus, are potentially capable of influencing many areas involved in perception, emotional processing, cognition, and behavior. Until recently, the dopamine hypothesis was the major pathophysiological theory of schizophrenia, based largely on the effectiveness of D2 antagonists in controlling the acute exacerbations of this disease.

Symptoms of schizophrenia, which typically emerge during adolescence or early adulthood, are usually classified as positive, negative or cognitive. Positive symptoms include hallucinations, delusions, suspiciousness, stereotyped thinking, somatic concern, unusual thought content or lack of judgment and insight. Negative symptoms are a group of deficits comprising blunted affect, emotional withdrawal, poor rapport, passive/apathetic social withdrawal, lack of spontaneity and flow of conversation, motor retardation or active social avoidance. Cognitive deficits, such as working memory, verbal memory, attention and executive function are also prominent features of the illness [2.2, 2.3].

Current atypical antipsychotics are efficacious primarily in the management of positive symptoms, yet have minimal effects on negative symptoms and cognitive deficits, besides being associated with significant side-effects. Efficacious treatments of both positive and negative symptoms and cognitive deficits are the highest unmet need in schizophrenia [2.3], [2.4].

First generation antipsychotics are effective but associated with significant incidence of extrapyramidal symptoms, whereas second-generation (atypical) antipsychotics have less propensity to cause extrapyramidal side-effects but are associated with an increased incidence and severity of metabolic syndrome.

A common antipsychotic drug for the treatment of schizophrenia is olanzapine (2-methyl-4-(4-methyl-1-piperazinyl)-10H-thieno[2.3-b][1.5]benzodiazepine). Olanzapine belongs to a drug class known as atypical antipsychotics. Other members of this class include paliperidone (3-[2-[4-(6-fluoro-1,2-benzisoxazol-3-yl)-1-piperidinyl]ethyl]-6,7,8,9-tetrahydro-9-hydroxy-2-methyl-), risperidone (3-[2-[4-(6-fluoro-1,2-benzisoxazol-3-yl)piperidino]ethyl]-2.methyl-6,7,8,9-tetrahydro-4H-pyrido-[1.2-a]pyrimidin-4-one), aripiprazole (7-{4-[4-(2,3-dichlorophenyl)piperazin-1-yl]butoxy}-3,4-dihydroquinolin-2(1*H*)-one), quetiapine (ethanol, 2-[2-(4-dibenzo[b,f]thiazepin-11-yl-1-piperazinyl)ethoxy]-) and ziprasidone (5-[2-[4-(1,2-benzisothiazol-3-yl)-1-piperazinyl]ethyl]-6-chloro-1,3-dihydro-2*H-*indol-2-one).

The most common drug for the treatment of schizophrenia is olanzapine. Olanzapine binds to alpha-1, dopamine, histamine, muscarinic and serotonin type 2 (5-HT2) receptors.
Olanzapine is approved for the treatment of schizophrenia, long term treatment of bipolar disorders and in combination with fluoxetine for the treatment of depressive episodes associated with bipolar disorders and for the treatment of resistant depression.
The treatment with antipsychotic drugs, such as with olanzapine, may lead to serious side effects. The Food and Drug Administration requires all atypical antipsychotics to include a warning about the risk of developing hyperglycemia and diabetes, both of which are factors in the metabolic syndrome. These effects may be related to the drug's ability to induce weight gain. There may be an enhanced risk of increased blood glucose levels and diabetes type II with olanzapine as well as the other antipsychotic medications in its class.

Therefore there is a need for new therapies with improved safety and tolerability profile over current atypical antipsychotics. For example, new treatments should not be associated with weight gain, extrapyramidal symptoms or effects on glucose and lipid metabolism [2.4, 2.5, 2.6].
The object of the present invention is a pharmaceutical combination comprising an atypical antipsychotic drug and a compound, which is an inhibitor on the GlyT1 for the treatment of negative and positive symptoms of schizophrenia without affecting/increasing the side-effect profile known from the treatment of atypical antipsychotics alone.
It has been shown that suitable GlyT1 inhibitors are compounds, disclosed in WO05/014563, for example the compounds of formula I: 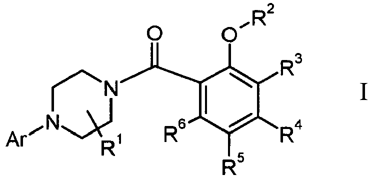 wherein
- Ar: is a substituted 6-membered heteroaryl group, containing one, two or three nitrogen atoms, and wherein the heteroaryl groups may be substituted by one or more substituents selected from the group consisting of halogen, (C₁-C₆)-alkyl or (C₁-C₆)-alkyl substituted by halogen;
- R¹: is hydrogen or (C₁-C₆)-alkyl;
- R²: is (C₁-C₆)-alkyl substituted by halogen,
- R³, R⁴ and R⁶: are independently from each other hydrogen, halogen, (C₁-C₆)-alkyl or (C₁-C₆)-alkoxy;
- R⁵: is SO₂R¹⁰.
- R¹⁰: is (C₁-C₆)-alkyl optionally subtituted by halogen,
or pharmaceutically acceptable acid addition salts thereof, as well as enantiomeric forms thereof.

The term "6-membered heteroaryl containing one, two or three nitrogen atoms" denotes a monovalent aromatic carbocyclic radical, for example pyridyl, pyrazinyl, pyrimidinyl, pyridazinyl or 1,3,5-triazinyl.

The term "halogen" denotes chlorine, iodine, fluorine and bromine.
The term "(C₁-C₆)-alkyl, substituted by halogen" denotes for example the following groups: CF₃, CHF₂, CH₂F, CH₂CF₃, CH₂CHF₂, CH₂CH₂F, CH₂CH₂CF₃, CH₂CH₂CH₂CF₃, CH₂CH₂Cl, CH₂CF₂CF₃, CH₂CF₂CHF₂, CF₂CHFCF₃, C(CH₃)₂CF₃, CH(CH₃)CF₃ or CH(CH₂F)CH₂F.

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methanesulfonic acid, p-toluenesulfonic acid and the like.

In more detail, the object of the present invention is a pharmaceutical combination comprising an atypical antipsychotic drug, selected from the group consisting of risperidone, paliperidone, olanzapine, aripiprazole, quetiapine or ziprasidone and a GlyT1 inhibitor selected from
rac-[4-(3-chloro-5-trifluoromethyl-pyridin-2-yl)-piperazin-1-yl]-[5-methanesulfonyl-2-(2,2,2-trifluoro-1-methyl-ethoxy)-phenyl]-methanone,
rac-[5-methanesulfonyl-2-(2,2,2-trifluoro-1-methyl-ethoxy)-phenyl]-[4-(5-trifluoromethyl-pyridin-2-yl)-piperazin-1-yl]-methanone,
rac-[4-(5-bromo-pyridin-2-yl)-piperazin-1-yl]-[5-methanesulfonyl-2-(2,2,2-trifluoro-1-methyl-ethoxy)-phenyl]-methanone,
rac-[4-(3-fluoro-5-trifluoromethyl-pyridin-2-yl)-piperazin-1-yl]-[5-methanesulfonyl-2-(2,2,2-trifluoro-1-methyl-ethoxy)-phenyl]-methanone,
rac-[5-methanesulfonyl-2-(2,2,2-trifluoro-1-methyl-ethoxy)-phenyl]-[4-(6-trifluoromethyl-pyridin-2-yl)-piperazin-1-yl]-methanone,
[5-methanesulfonyl-2-((S or R)-2,2,2-trifluoro-1-methyl-ethoxy)-phenyl]-[4-(5-trifluoromethyl-pyridin-2-yl)-piperazin-1-yl]-methanone,
[5-methanesulfonyl-2-((R or S)-2,2,2-trifluoro-1-methyl-ethoxy)-phenyl]-[4-(5-trifluoromethyl-pyridin-2-yl)-piperazin-1-yl]-methanone,
[4-((3-fluoro-5-trifluoromethyl-pyridin-2-yl)-piperazin-1-yl]-[5-methanesulfonyl-2-((S)-2,2,2-trifluoro-1-methyl-ethoxy)-phenyl]-methanone or
[4-((3-fluoro-5-trifluoromethyl-pyridin-2-yl)-piperazin-1-yl]-[5-methanesulfonyl-2-(2,2,2-trifluoro-1,1-dimethyl-ethoxy)-phenyl]-methanone.

More specifically, the invention comprises a pharmaceutical combination of an atypical antipsychotic drug, selected from the group consisting of risperidone, paliperidone, olanzapine, aripiprazole, quetiapine or ziprasidone and the GlyT1 inhibitor[4-(3-fluoro-5-trifluoromethyl-pyridin-2-yl)-piperazin-1-yl]-[5-methanesulfonyl-2-(2,2,2-trifluoro-1-methyl-ethoxy)-phenyl]-methanone.

The pharmaceutical combination as mentioned above comprises an atypical antipsychotic drug and a GlyT1 inhibitor of formula I for the treatment of positive and negative symptoms in schizophrenia.

A further object of the present invention is the use of a pharmaceutical combination comprising an atypical antipsychotic drug, selected from the group consisting of risperidone, paliperidone, olanzapine, aripiprazole, quetiapine or ziprasidone and a GlyT1 inhibitor selected from
rac-[4-(3-chloro-5-trifluoromethyl-pyridin-2-yl)-piperazin-1-yl]-[5-methanesulfonyl-2-(2,2,2-trifluoro-1-methyl-ethoxy)-phenyl]-methanone,
rac-[5-methanesulfonyl-2-(2,2,2-trifluoro-1-methyl-ethoxy)-phenyl]-[4-(5-trifluoromethyl-pyridin-2-yl)-piperazin-1-yl]-methanone,
rac-[4-(5-bromo-pyridin-2-yl)-piperazin-1-yl]-[5-methanesulfonyl-2-(2,2,2-trifluoro-1-methyl-ethoxy)-phenyl]-methanone,
rac-[4-(3-fluoro-5-trifluoromethyl-pyridin-2-yl)-piperazin-1-yl]-[5-methanesulfonyl-2-(2,2,2-trifluoro-1-methyl-ethoxy)-phenyl]-methanone,
rac-[5-methanesulfonyl-2-(2,2,2-trifluoro-1-methyl-ethoxy)-phenyl]-[4-(6-trifluoromethyl-pyridin-2-yl)-piperazin-1-yl]-methanone,
[5-methanesulfonyl-2-((S or R)-2,2,2-trifluoro-1-methyl-ethoxy)-phenyl]-[4-(5-trifluoromethyl-pyridin-2-yl)-piperazin-1-yl]-methanone,
[5-methanesulfonyl-2-((R or S)-2,2,2-trifluoro-1-methyl-ethoxy)-phenyl]-[4-(5-trifluoromethyl-pyridin-2-yl)-piperazin-1-yl]-methanone,
[4-((3-fluoro-5-trifluoromethyl-pyridin-2-yl)-piperazin-1-yl]-[5-methanesulfonyl-2-((S)-2,2,2-trifluoro-1-methyl-ethoxy)-phenyl]-methanone or
[4-((3-fluoro-5-trifluoromethyl-pyridin-2-yl)-piperazin-1-yl]-[5-methanesulfonyl-2-(2,2,2-trifluoro-1,1-dimethyl-ethoxy)-phenyl]-methanone
for the treatment of positive and negative symptoms in schizophrenia.

In more detail, a further object of the present invention is the use of a pharmaceutical combination comprising an atypical antipsychotic drug, selected from the group consisting of risperidone, paliperidone, olanzapine, aripiprazole, quetiapine or ziprasidone and the GlyT1 inhibitor[4-(3-fluoro-5-trifluoromethyl-pyridin-2-yl)-piperazin-1-yl]-[5-methanesulfonyl-2-(2,2,2-trifluoro-1-methyl-ethoxy)-phenyl]-methanone for the treatment of positive and negative symptoms in schizophrenia.

One embodiment of the invention is the compound 4-(3-fluoro-5-trifluoromethyl-pyridin-2-yl)-piperazin-1-yl]-[5-methanesulfonyl-2-((S)-2,2,2-trifluoro-1-methyl-ethoxy)-phenyl]-methanone (RG1678).

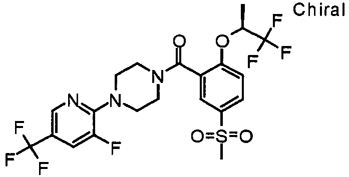

A preferred combination comprises RG1678 and risperidone or olanzapine.
RG1678 selectively inhibits GLYT1, a transporter known to control brain extracellular levels of glycine in the vicinity of NMDA-R [2.7, 2.8]. Increase of glycine leads to a positive modulation ofNMDA-R synaptic activity, thought to be deficient and/or function sub-optimally in the central nervous system of schizophrenic patient [2.8, 2.9, 2.10]. Advantages over the existing antipsychotic therapies include the potential for improving negative symptoms and cognitive deficits which consequently may lead to better social and functional outcome as well as an improved tolerability profile, being devoid of the D2/5-HT2A class liabilities.

### NMDA receptor hypofunction hypothesis and GlyT1 inhibition concept

A growing body of evidence, underscoring the involvement of NMDA-R hypofunction in the pathophysiology of schizophrenia, has been evolving over the past 18 years from studies in normal individuals and animals as well as from genetic analysis and patients with schizophrenia [2.4, 2.10, 2.11]. Thus therapeutic intervention aimed to increase NMDA receptor functioning are expected to have a significant benefit on the mental health of schizophrenic patients [2.4, 2.5, 2.9, 2.10].

As glycine is an obligatory co-agonist at the NMDA-R complex [2.10], one strategy to enhance NMDA-R mediated neurotransmission is to elevate extracellular levels of glycine in the local microenvironment of synaptic NMDA receptors by inhibiting the glycine transporter 1 (GLYT1), the only sodium-chloride dependent glycine transporter in the forebrain where it is co-expressed with the NMDA-R and responsible for glycine removal from the synaptic cleft [2.9, 2.10].

Several preclinical reports provide support for this approach as do recent findings demonstrating the regulation of dopaminergic neurotransmission by GLYT1 inhibition [2.9, 2.12, 2.13].

### Clinical trials of glycine, D-serine and sarcosine as add-on

Additional support for this approach in the treatment of schizophrenia and psychosis comes from clinical studies where glycine and D-serine (co-agonists at the glycine site of NMDA-R) and sarcosine (a prototypical weak GLYT1 inhibitor) improved positive, negative and cognitive symptoms in schizophrenic patients, when added to conventional therapy [2.14, 2.15, 2.16, 2.17, 2.18]

A behavioural assay was developed for rapid identification of in vivo active compounds (D. Alberati et al 2010; Pharmacol Biochem, Behav, accepted for publication). This method is based on the induction of hyperlocomotion in mice due to blockade ofNMDA receptor through administration of L-687,414 ((3*R*,4*R*)-3-amino-1-hydroxy-4-methyl pyrrolidin-2-one, a partial agonist at the glycine site of the NMDA receptor complex. It was shown that glycine and GlyT1 inhibitors dose-dependently blocked hyperlocomotion induced by L-687,414 most likely via synaptic glycine elevation (induced by either direct glycine administration or GlyT1 inhibition) which in turn can displace L-687,414 from the NMDA receptor binding site and, thus, normalize behavioral alteration induced by NMDA receptor blockade. In addition it was observed that, whereas psychoactive drugs like antidepressants, benzodiazepines or analgesics failed to prevent the hyperlocomotion induced by L-687,414, antipsychotic drugs (haloperidol, olanzapine, risperidone and aripiprazole) were all effective in preventing this behavioral effect in a dose-dependant manner. Therefore, this novel behavioral assay robustly and reliably detects the in vivo activity of GlyT1 inhibitors and antipsychotic drugs.
In light of the clinical studies which have demonstrated the efficacy of glycine, D-serine (co-agonists at the glycine-site of the NMDA receptor) and sarcosine (a weak GLYT1 inhibitor) in improving positive, negative and cognitive symptoms in schizophrenic patients, when added to conventional therapy, the effect of RG1678 in combination with antipsychotics was investigated in mice challenged with L-687,414.

### MATERIALS AND METHODS

### Drugs

RG1678, [4-(3-Fluoro-5-trifluoromethyl-pyridin-2-yl)-piperazin-1-yl]-[5-methanesulfonyl-2-((*S*)-2,2,2 trifluoro-1-methyl-ethoxy)-phenyl]-methanone (WO05/014563) and L-687,414, ((3*R*,4*R*)-3-amino-1-hydroxy-4-methyl pyrrolidin-2-one (Tetrahedron Letters, Vol. 49, issue 42, 2008, 6079-6080) and olanzapine were synthesized according to known methods by the Medicinal Chemistry Department of F. Hoffmann-La Roche, and risperidone was purchased from Sigma. All drugs were dissolved in H₂O/0.3 % Tween 80 and administered orally in a volume of 10 ml/kg body weight.

### Animals

Male NMRI mice (20 - 30 g) supplied from Iffa Credo, Lyon, France, were housed in a vivarium at controlled temperature (20 -22 °C) and a 12 hr light/dark cycle (lights on 6:00 a.m. Animals were allowed *ad libitum* access to food and water. The experimental procedures used in the present study received prior approval from the City of Basel Cantonal Animal Protection Committee based on adherence to federal and local regulations. Behavioral experiments were conducted during the hours of 8:00 a.m. and 2:00 p.m.

### Reversal of L-687,414-induced hyperlocomotion in mice

A computerized Digiscan 16 Animal Activity Monitoring System (Omnitech Electronics, Columbus, Ohio) was used to quantify locomotor activity. Data were obtained simultaneously from eight Digiscan activity chambers placed in a soundproof room with a 12 hr light/dark cycle. Experiments were performed during the light phase between 06:30 a.m. and 5:00 p.m. Each activity monitoring chamber consisted of a Plexiglas box (41 x 41 x 28 cm; W x L x H) with sawdust bedding on the floor surrounded by invisible horizontal and vertical infrared sensor beams. The chambers were divided by a Plexiglas cross providing each mouse with 20 x 20 cm of moving space. Two animals per box were monitored simultaneously. Chambers were connected to a Digiscan Analyzer linked to a computer that constantly collected the beam status information. The activity detector operates by counting the number of times the beams change from uninterrupted to interrupted status or vice-versa. Records of photocell beam interruptions for individual animals were taken every five minutes over the duration of the experimental session. Mice were first treated with RG1678 at different doses or at a fixed low dose administered p.o. and, 30 minutes later, treated with an antipsychotic at different doses or at a fixed low dose administered p.o. Fifteen minutes after the antipsychotic treatment mice received a s.c. injection of 50 mg/kg of L-687,414. Mice were then transferred from their home cages to the recording chambers for a 15-min habituation phase allowing free exploration of the new environment. Horizontal activity was then recorded for a 60-min time period. The horizontal activity value for each group of animals at a given dose of RG1678 alone or in combination with an antipsychotic (y1) was expressed as % of L-687,414-induced hyperlocomotion and calculated according to the equation (((y1 - vehicle horizontal activity)/(L-687,414 horizontal activity-vehicle horizontal activity)) x 100). For dose-response experiments, the horizontal activity value for each group of animal at a given dose of GlyT1 inhibitor or antipsychotics (y1) was expressed as a percent of L-687,414-induced hyperlocomotion and calculated according to the equation (((y1 - vehicle horizontal activity)/(L-687,414 horizontal activity-vehicle horizontal activity))x100). ID₅₀ values, defined as doses of each compound producing 50% inhibition of L-687,414-induced hyperlocomotion, were calculated by linear regression analysis of the dose-response data using an Excel-based computer curve-fitting program.

### RESULTS

In all experiments the 50 mg/kg dose of L-687,414 was used, as it was previously found to trigger a high and reliable behavioral activation in mice as compared to vehicle-treated animals. RG1678, risperidone and olanzapine and all dose-dependently reversed hyperlocomotion induced by L-687,414 in mice.

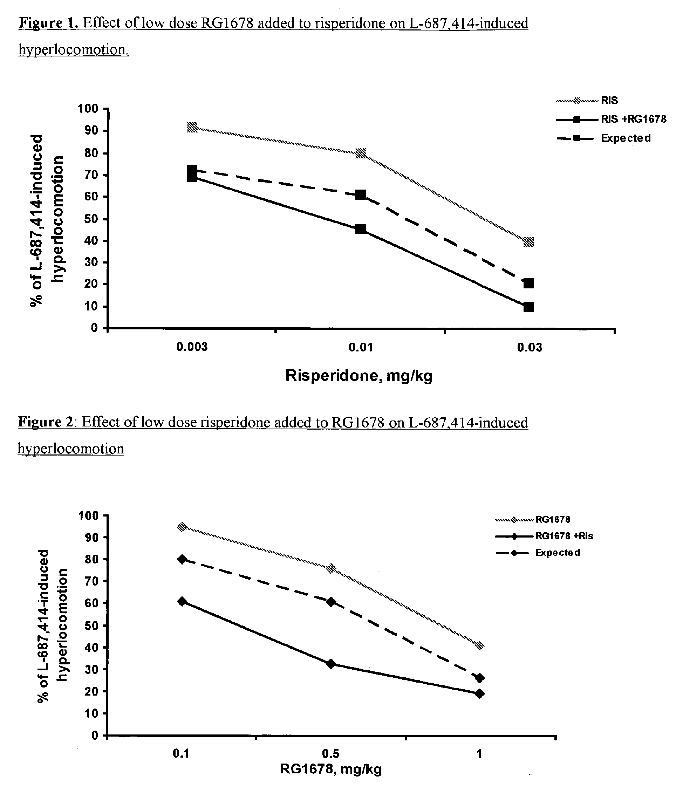

### Figure 1. Effect of low dose RG1678 added to risperidone on L-687,414-induced hyperlocomotion.

Male NMRI mice were treated with RG1678 0.6 mg/kg p.o., followed 30' later by risperidone p.o. in doses ranging from 0.003 to 0.3 mg/kg. After 15' a subcutaneous injection of 50 mg/kg of L-687,414 was given. Controls animals received vehicle only or vehicle and L-687,414. Recording of motor activity started 15 min later and lasted 1 hour. Data are means based on 8 animals per group. Full grey line: risperidone alone; full black line: risperidone plus RG1678 0.6 mg/kg; dashed black line: expected effect of risperidone and RG1678 0.6 mg/kg based on a 19% reversal of hyperlocomotion induced by RG1678 alone. The ED₅₀ of risperidone alone was 0.023 mg/kg.

### Result

When a low dose of RG1678 (0.6 mg/kg) was added to risperidone (dose-response curve) the efficacy of this antipsychotic was enhanced to a level higher than the one expected on the base of the risperidone and RG1678 alone (Figure 1).

### Figure 2: Effect of low dose risperidone added to RG1678 on L-687.414-induced hyperlocomotion

Male NMRI mice were treated with RG1678 in doses ranging from 0.1 to 1 mg/kg p.o., followed 30' later by risperidone 0.005 mg/kg p.o.. After 15' a subcutaneous injection of 50 mg/kg of L-687,414 was given. Controls animals received vehicle only or vehicle and L-687,414. Recording of motor activity started 15 min later and lasted 1 hour. Data are means based on 8 animals per group. Full grey line: RG1678 alone; full black line: RG1678 plus risperidone; dashed black line: expected effect of RG1678 and risperidone 0.005 mg/kg based on a 15 % reversal of hyperlocomotion induced by risperidone alone. The ED₅₀ of RG1678 alone was 0.76 mg/kg. (Figure 2).

### Result

The same effect as for figure 1 was observed when low dose of risperidone (0.005 mg/kg) was added to RG1678 (dose-response)

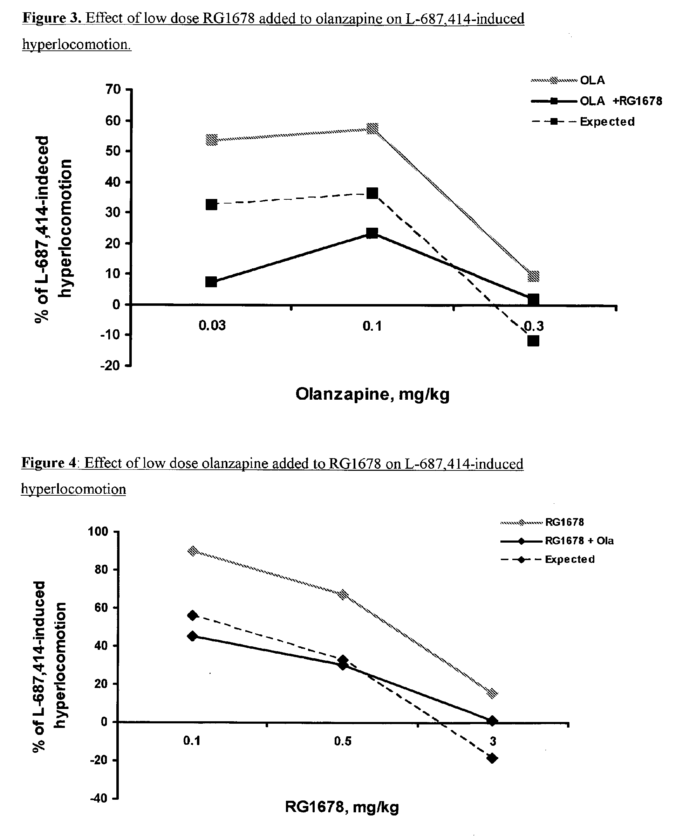

### Figure 3. Effect of low dose RG1678 added to olanzapine on L-687,414-induced hyperlocomotion.

Male NMRI mice were treated with RG1678 0.6 mg/kg p.o., followed 30' later by olanzapine p.o. in doses ranging from 0.003 to 0.3 mg/kg. After 15' a subcutaneous injection of 50 mg/kg of L-687,414 was given. Controls animals received vehicle only or vehicle and L-687,414. Recording of motor activity started 15 min later and lasted 1 hour. Data are means based on 8 animals per group. Full grey line: olanzapine alone; full black line: olanzapine plus RG1678 0.6 mg/kg; dashed black line: expected effect of olanzapine and RG1678 0.6 mg/kg based on a 21% reversal of hyperlocomotion induced by RG1678 alone. The ED₅₀ of olanzapine alone was 0.06 mg/kg.

### Result

Similar effect were observed when low dose of RG1678 (0.6 mg/kg) was added to olanzapine (dose-response curve), where again the efficacy of this antipsychotic was enhanced to a level higher than the one expected on the base of the olanzapine and RG1678 alone (Figure 3).

### Figure 4: Effect of low dose olanzapine added to RG1678 on L-687,414-induced hyperlocomotion

Male NMRI mice were treated with RG1678 in doses ranging from 0.1 to 3 mg/kg p.o., followed 30' later by olanzapine 0.05 mg/kg p.o.. After 15' a subcutaneous injection of 50 mg/kg of L-687,414 was given. Controls animals received vehicle only or vehicle and L-687,414. Recording of motor activity started 15 min later and lasted 1 hour. Data are means based on 8 animals per group. Full grey line: RG1678 alone; full black line: RG1678 plus olanzapine; dashed black line: expected effect of RG1678 and olanzapine 0.05mg/kg based on a 34% reversal of hyperlocomotion induced by olanzapine alone. The ED₅₀ of RG1678 alone was 0.83 mg/kg.

### Result

When low dose of olanzapine (0.05 mg/kg) were added to RG1678 (dose-response) a clear enhanced efficacy of RG1678 could be detected. In this combination, however the effect observed was closed to the one expected based on the efficacy of olanzapine and RG1678 alone (Figure 4).

Altogether these behavioral pharmacology studies do support the notion that low doses of RG1678 combined with antipsychotic drugs enhances their efficacy. The efficacy of the combination of a GlyT1 inhibitor and an atypical antipsychotic drug has been increased if compared the efficacy of active components alone.

The atypical antipsychotic drugs, for example olanzapine, and a compounds of formula I as well as the pharmaceutically acceptable salt can be used as medicaments, e.g. in the form of pharmaceutical preparations. The pharmaceutical preparations can be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions.

The compounds of formula I can be processed with pharmaceutically inert, inorganic or organic carriers for the production of pharmaceutical preparations. Lactose, corn starch, cellulose or derivatives thereof, talc, stearic acids or its salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are however usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing an atypical antipsychotic drug, for example olanzapine, and a compound of formula I or a pharmaceutically acceptable salt thereof and a therapeutically inert carrier are also an object of the present invention, as is a process for their production, which comprises bringing one or more compounds of formula I and the antipsychotic compound and/or pharmaceutically acceptable acid addition salts and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration the dosage for adults can vary from about 0.01 mg to about 1000 mg per day of the antipsychotic drug and a compound of general formula I or of the corresponding amount of a pharmaceutically acceptable salt thereof. The daily dosage may be administered as single dose or in divided doses and, in addition, the upper limit can also be exceeded when this is found to be indicated. Normally, the primary atypical antipsychotics are administered in a dose range according to the approved local prescribing information.

**Tablet Formulation (Wet Granulation)**

| Item | Ingredients | mg/tablet | | | |
|---|---|---|---|---|---|
| | | 5 mg | 25 mg | 100 mg | 500 mg |
| 1. | Active compound | 5 | 25 | 100 | 500 |
| 2. | Lactose Anhydrous DTG | 125 | 105 | 30 | 150 |
| 3. | Sta-Rx 1500 | 6 | 6 | 6 | 30 |
| 4. | Microcrystalline Cellulose | 30 | 30 | 30 | 150 |
| 5. | Magnesium Stearate | 1 | 1 | 1 | 1 |
| | Total | 167 | 167 | 167 | 831 |

### Manufacturing Procedure

1. Mix items 1, 2, 3 and 4 and granulate with purified water.
2. Dry the granules at 50°C.
3. Pass the granules through suitable milling equipment.
4. Add item 5 and mix for three minutes; compress on a suitable press.

**Capsule Formulation**

| Item | Ingredients | mg/capsule | | | |
|---|---|---|---|---|---|
| | | 5 mg | 25 mg | 100 mg | 500 mg |
| 1. | Active compound | 5 | 25 | 100 | 500 |
| 2. | Hydrous Lactose | 159 | 123 | 148 | --- |
| 3. | Corn Starch | 25 | 35 | 40 | 70 |
| 4. | Talc | 10 | 15 | 10 | 25 |
| 5. | Magnesium Stearate | 1 | 2 | 2 | 5 |
| | Total | 200 | 200 | 300 | 600 |

### Manufacturing Procedure

1. Mix items 1, 2 and 3 in a suitable mixer for 30 minutes.
2. Add items 4 and 5 and mix for 3 minutes.
3. Fill into a suitable capsule.

**Olanzapine Tablet Formulation**

| Item | Ingredients | mg/capsule | | | |
|---|---|---|---|---|---|
| | | 2.5 mg | 7.5 mg | 15.0 mg | 20.0 mg |
| 1. | Olanzapine | 2.5 | 7.5 | 15.0 | 20.0 |
| 2. | Lactose monohydrate | 89.0 | 84.0 | 76.5 | 71.5 |
| 3. | Hyprolose | 7.5 | 7.5 | 7.5 | 7.5 |
| 4. | Crospovidon | 4.5 | 4.5 | 4.5 | 4.5 |
| 5. | Microcrystalline Cellulose | 45.0 | 45.0 | 45.0 | 45.0 |
| 6. | Magnesiumstearate | 1.5 | 1.5 | 1.5 | 1.5 |
| | Total | 150.0 | 150.0 | 150.0 | 150.0 |

### Manufacturing Procedure

1. Mix items 1 to 5 and granulate with purified water.
2. Dry the granules at 50°C.
3. Pass the granules through suitable milling equipment.
4. Add item 6 and mix for three minutes; compress on a suitable press.

**Combination formulation**

| Item | Ingredients | mg/capsule | | |
|---|---|---|---|---|
| | GlyT1 inhib/antipsych. | 5.0/2.5 | 25.0/2.5 | 100.0/15.0 mg |
| 1. | Glyt1 inh. | 5.0 | 25.0 | 100.0 |
| 2. | Olanzapine | 2.5 | 2.5 | 15.0 |
| 3. | Lactose monohydrate | 166.25 | 146.25 | 58.75 |
| 4. | Povidon K30 | 12.5 | 12.5 | 12.5 |
| 5. | Croscarmellose Sodium | 7.5 | 7.5 | 7.5 |
| 6. | Microcrystalline Cellulose | 50.0 | 50.0 | 50.0 |
| 7. | Magnesiumstearate | 1.25 | 1.25 | 1.25 |
| 8. | Talc | 5.0 | 5.0 | 5.0 |
| | Total | 250.0 | 250.0 | 250.0 |

### Manufacturing Procedure

1. Mix items 1 to 6 and granulate with purified water.
2. Dry the granules at 50°C.
3. Pass the granules through suitable milling equipment.
4. Add item 7 and 8 and mix for three minutes; compress on a suitable press.

### Literature

2.1. Jablensky A. Epidemiology of schizophrenia. In: Gelder MG, Lopez-Ibor Jr JJ, Andreasen NC, eds. New Oxford Textbook of Psychiatry, Oxford, GB: Oxford University Press; 2000: 585-598.
2.2. American psychiatry association. DSM-IV Diagnostic and statistical manual of mental disorders. 4th ed. Washington DC: APA, 1994.
2.3. Stip E et al. On the trail of a cognitive enhancer for the treatment of schizophrenia. Prog Neuropsychopharmacol Biol Psychiatry. 2005; 29:219-232 and citations herein.
2.4. Sanger DJ The search for novel antipsychotics: pharmacological and molecular targets. Expert Opin Ther Targets. 2004; 8:631-641.
2.5. Chavez-Noriega LE et al. Novel potential therapeutics for schizophrenia: focus on the modulation of metabotropic glutamate receptor function. Curr Neuropharmacol. 2005; 3:9-34 and citations herein.
2.6. Bergman RN et al. Atypical antipsychotics and glucose homeostasis J Clin Psychiatry. 2005; 66:504-514.
2.7. Cubelos B et al. Localization of the GLYT1 glycine transporter at glutamatergic synapses in the rat brain. Cereb Cortex. 2005; 15:448-459
2.8. Eulenburg V et al. Glycine transporters: essential regulators of neurotransmission. Trends Biochem Sci. 2005; 30:325-333.
2.9. Sur C et al. The therapeutic potential of glycine transporter-1 inhibitors. Expert Opin Investig Drugs. 2004; 13:515-521.
2.10. Millan MJ. N-Methyl-D-aspartate receptors as a target for improved antipsychotic agents: novel insights and clinical perspectives. Psychopharmacology. 2005; 179:30-53.
2.11. Harrison P et al. Schizophrenia genes, gene expression and neuropathology: on the matter of their convergence. Mol Psychiatry. 2005; 10:40-68.
2.12. Lechner SM. Glutamate-based therapeutic approaches: inhibitors of glycine transport. Curr Op Pharmacol. 2006; 6:1-7.
2.13. Javitt DC et al. Modulation of striatal dopamine release by glycine transport inhibitors. Neuropsychopharmacology. 2005; 30: 649-656.
2.14. Heresco-Levy U et al. high-dose glycine added to olanzapine and risperidone for the treatment of schizophrenia. Biol Psychiatry. 2004; 15:165-171.
2.15. Tsai G et al. D-serine added to antipsychotics for the treatment of schizophrenia. Biol Psychiatry. 1998; 44:1081-1089.
2.16. Heresco-levy U et al. D-serine efficacy as add-on pharmacotherapy to risperidone and olanzapine for treatment-refractory schizophrenia. Biol Psychiatry. 2005; 15:577-585.
2.17. Tsai G et al. Glycine transporter I inhibitor, N-methylglycine (sarcosine) added to antipsychotics for the treatment of schizophrenia. Biol Psychiatry. 2004; 55: 452-456.
2.18. Lane HY et al. Sarcosine and D-serine add-on treatment for acute exacerbation of schizophrenia: a randomized, double-blind, placebocontrolled study. Arch Gen Psychiatry. 2005; 62: 1196-1204.

## Claims

1. A pharmaceutical combination comprising an atypical antipsychotic drug and a GlyT1 receptor antagonist of formula 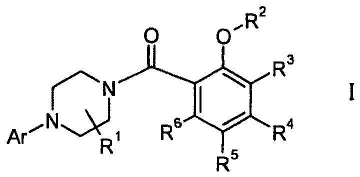 wherein
Ar is a substituted 6-membered heteroaryl group, containing one, two or three nitrogen atoms, and wherein the heteroaryl groups may be substituted by one or more substituents selected from the group consisting of halogen, (C₁-C₆)-alkyl or (C₁-C₆)-alkyl substituted by halogen;
R¹ is hydrogen or (C₁-C₆)-alkyl;
R² is (C₁-C₆)-alkyl substituted by halogen,
R³, R⁴ and R⁶ are independently from each other hydrogen, halogen, (C₁-C₆)-alkyl or (C₁-C₆)-alkoxy;
R⁵ is SO₂R¹⁰;
R¹⁰ is (C₁-C₆)-alkyl optionally substituted by halogen,
and pharmaceutically acceptable acid addition salts thereof, as well as enantiomeric forms thereof.

2. A pharmaceutical combination according to claim 2 comprising an atypical antipsychotic drug, selected from the group consisting of risperidone, paliperidone, olanzapine, aripiprazole, quetiapine or ziprasidone and a GlyT1 receptor antagonist selected from
rac-[4-(3-chloro-5-trifluoromethyl-pyridin-2-yl)-piperazin-1-yl]-[5-methanesulfonyl-2-(2,2,2-trifluoro-1-methyl-ethoxy)-phenyl]-methanone,
rac-[5-methanesulfonyl-2-(2,2,2-trifluoro-1-methyl-ethoxy)-phenyl]-[4-(5-trifluoromethyl-pyridin-2-yl)-piperazin-1-yl]-methanone,
rac-[4-(5-bromo-pyridin-2-yl)-piperazin-1-yl]-[5-methanesulfonyl-2-(2,2,2-trifluoro-1-methyl-ethoxy)-phenyl]-methanone,
rac-[4-(3-fluoro-5-trifluoromethyl-pyridin-2-yl)-piperazin-1-yl]-[5-methanesulfonyl-2-(2,2,2-trifluoro-1-methyl-ethoxy)-phenyl]-methanone,
rac-[5-methanesulfonyl-2-(2,2,2-trifluoro-1-methyl-ethoxy)-phenyl]-[4-(6-trifluoromethyl-pyridin-2-yl)-piperazin-1-yl]-methanone,
[5-methanesulfonyl-2-((S or R)-2,2,2-trifluoro-1-methyl-ethoxy)-phenyl]-[4-(5-trifluoromethyl-pyridin-2-yl)-piperazin-1-yl]-methanone,
[5-methanesulfonyl-2-((R or S)-2,2,2-trifluoro-1-methyl-ethoxy)-phenyl]-[4-(5-trifluoromethyl-pyridin-2-yl)-piperazin-1-yl]-methanone,
[4-((3-fluoro-5-trifluoromethyl-pyridin-2-yl)-piperazin-1-yl]-[5-methanesulfonyl-2-((S)-2,2,2-trifluoro-1-methyl-ethoxy)-phenyl]-methanone or
[4-((3-fluoro-5-trifluoromethyl-pyridin-2-yl)-piperazin-1-yl]-[5-methanesulfonyl-2-(2,2,2-trifluoro-1,1-dimethyl-ethoxy)-phenyl]-methanone.

3. A pharmaceutical combination according to claim 2 comprising an atypical antipsychotic drug, selected from the group consisting of of risperidone, paliperidone, olanzapine, aripiprazole, quetiapine or ziprasidone and the GlyT1 receptor antagonist [4-(3-fluoro-5-trifluoromethyl-pyridin-2-yl)-piperazin-1-yl]-[5-methanesulfonyl-2-(2,2,2-trifluoro-1-methyl-ethoxy)-phenyl]-methanone.

4. A pharmaceutical combination comprising an atypical antipsychotic drug selected from the group consisting of olanzapine or risperidone and a GlyT1 receptor antagonist which is 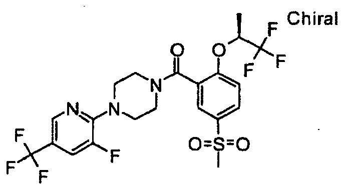 and pharmaceutically acceptable acid addition salts thereof, as well as enantiomeric forms thereof.

5. A pharmaceutical combination according to claim 1 comprising an atypical antipsychotic drug and a GlyT1 receptor antagonist for use in the treatment of positive and negative symptoms in schizophrenia.

6. The use of a pharmaceutical combination comprising an atypical antipsychotic drug and a GlyT1 receptor antagonist of formula 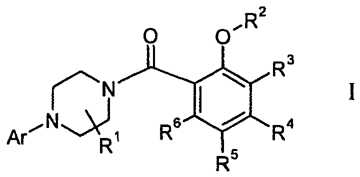 wherein
Ar is a substituted 6-membered heteroaryl group, containing one, two or three nitrogen atoms, and wherein the heteroaryl groups may be substituted by one or more substituents selected from the group consisting of halogen, (C₁-C₆)-alkyl or (C₁-C₆)-alkyl substituted by halogen;
R¹ is hydrogen or (C₁-C₆)-alkyl;
R² is (C₁-C₆)-alkyl substituted by halogen,
R³, R⁴ and R⁶ are independently from each other hydrogen, halogen, (C₁-C₆)-alkyl or (C₁-C₆)-alkoxy;
R⁵ is SO₂R¹⁰;
R¹⁰ is (C₁-C₆)-alkyl optionally subtituted by halogen,
and pharmaceutically acceptable acid addition salts thereof, as well as enantiomeric forms thereof for the preparation of a medicament for the treatment of positive and negative symptoms in schizophrenia.

7. The use of a pharmaceutical combination according to claim 6 comprising an atypical antipsychotic drug, selected from the group consisting of of risperidone, paliperidone, olanzapine, aripiprazole, quetiapine or ziprasidone and a GlyT1 receptor antagonist selected from
rac-[4-(3-chloro-5-trifluoromethyl-pyridin-2-yl)-piperazin-1-yl]-[5-methanesulfonyl-2-(2,2,2-trifluoro-1-methyl-ethoxy)-phenyl]-methanone,
rac-[5-methanesulfonyl-2-(2,2,2-trifluoro-1-methyl-ethoxy)-phenyl]-[4-(5-trifluoromethyl-pyridin-2-yl)-piperazin-1-yl]-methanone,
rac-[4-(5-bromo-pyridin-2-yl)-piperazin-1-yl]-[5-methanesulfonyl-2-(2,2,2-trifluoro-1-methyl-ethoxy)-phenyl]-methanone,
rac-[4-(3-fluoro-5-trifluoromethyl-pyridin-2-yl)-piperazin-1-yl]-[5-methanesulfonyl-2-(2,2,2-trifluoro-1-methyl-ethoxy)-phenyl]-methanone,
rac-[5-methanesulfonyl-2-(2,2,2-trifluoro-1-methyl-ethoxy)-phenyl]-[4-(6-trifluoromethyl-pyridin-2-yl)-piperazin-1-yl]-methanone,
[5-methanesulfonyl-2-((S or R)-2,2,2-trifluoro-1-methyl-ethoxy)-phenyl]-[4-(5-trifluoromethyl-pyridin-2-yl)-piperazin-1-yl]-methanone,
[5-methanesulfonyl-2-((R or S)-2,2,2-trifluoro-1-methyl-ethoxy)-phenyl]-[4-(5-trifluoromethyl-pyridin-2-yl)-piperazin-1-yl]-methanone,
[4-((3-fluoro-5-trifluoromethyl-pyridin-2-yl)-piperazin-1-yl]-[5-methanesulfonyl-2-((S)-2,2,2-trifluoro-1-methyl-ethoxy)-phenyl]-methanone or
[4-((3-fluoro-5-trifluoromethyl-pyridin-2-yl)-piperazin-1-yl]-[5-methanesulfonyl-2-(2,2,2-trifluoro-1,1-dimethyl-ethoxy)-phenyl]-methanone
for the preparation of a medicament for the treatment of positive and negative symptoms in schizophrenia.

8. The use of a pharmaceutical combination according to claim 7 comprising an atypical antipsychotic drug, selected from the group consisting of of risperidone, paliperidone, olanzapine, aripiprazole, quetiapine or ziprasidone and the GlyT1 receptor antagonist [4-(3-fluoro-5-trifluoromethyl-pyridin-2-yl)-piperazin-1-yl]-[5-methanesulfonyl-2-(2,2,2-trifluoro-1-methyl-ethoxy)-phenyl]-methanone for the preparation of a medicament for the treatment of positive and negative symptoms in schizophrenia.

9. The use of a pharmaceutical combination according to claim 8, comprising an atypical antipsychotic drug selected from the group consisting of olanzapine or risperidone and a GlyT1 receptor antagonist which is 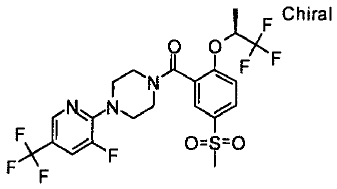 and pharmaceutically acceptable acid addition salts thereof, as well as enantiomeric forms thereof.

## Patentansprüche

1. Eine pharmazeutische Kombination, umfassend einen atypischen antipsychotischen Arzneistoff und einen GlyT1-Rezeptor-Antagonisten der Formel 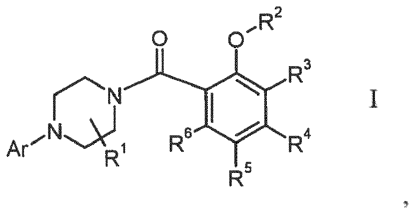 wobei
Ar ein substituierter 6-gliedriger Heteroarylrest ist, der ein, zwei oder drei Stickstoffatome enthält, und wobei die Heteroarylreste mit einem oder mehreren Substituenten substituiert sein können, ausgewählt aus der Gruppe bestehend aus Halogen, (C₁-C₆)-Alkyl oder (C₁-C₆)-Alkyl, substituiert mit Halogen;
R¹ oder (C₁-C₆)-Alkyl ist;
R² (C₁-C₆)-Alkyl, substituiert mit Halogen, ist,
R³, R⁴ und R⁶ unabhängig voneinander Wasserstoff, Halogen, (C₁-C₆)-Alkyl oder (C₁-C₆)-Alkoxy sind;
R⁵ SO₂R¹⁰ ist;
R¹⁰ (C₁-C₆)-Alkyl, gegebenenfalls substituiert mit Halogen, ist,
und pharmazeutisch verträgliche Säureadditionssalze davon sowie enantiomere Formen davon.

2. Eine pharmazeutische Kombination gemäß Anspruch 2, umfassend einen atypischen antipsychotischen Arzneistoff, ausgewählt aus der Gruppe bestehend aus Risperidon, Paliperidon, Olanzapin, Aripiprazol, Quetiapin oder Ziprasidon, und einen GlyT1-Rezeptor-Antagonisten, ausgewählt aus
rac-[4-(3-Chlor-5-trifluormethyl-pyridin-2-yl)-piperazin-1-yl]-[5-methansulfonyl-2-(2,2,2-trifluor-1-methyl-ethoxy)-phenyl]-methanon,
rac-[5-Methansulfonyl-2-(2,2,2-trifluor-1-methyl-ethoxy)-phenyl]-[4-(5-trifluormethyl-pyridin-2-yl)-piperazin-1-yl]-methanon,
rac-[4-(5-Brom-pyridin-2-yl)-piperazin-1-yl]-[5-methansulfonyl-2-(2,2,2-trifluor-1-methyl-ethoxy)-phenyl]-methanon,
rac-[4-(3-Fluor-5-trifluormethyl-pyridin-2-yl)-piperazin-1-yl]-[5-methansulfonyl-2-(2,2,2-trifluor-1-methyl-ethoxy)-phenyl]-methanon,
rac-[5-Methansulfonyl-2-(2,2,2-trifluor-1-methyl-ethoxy)-phenyl]-[4-(6-trifluormethyl-pyridin-2-yl)-piperazin-1-yl]-methanon,
[5-Methansulfonyl-2-((S oder R)-2,2,2-trifluor-1-methyl-ethoxy)-phenyl]-[4-(5-trifluormethyl-pyridin-2-yl)-piperazin-1-yl]-methanon,
[5-Methansulfonyl-2-((R oder S)-2,2,2-trifluor-1-methyl-ethoxy)-phenyl]-[4-(5-trifluormethyl-pyridin-2-yl)-piperazin-1-yl]-methanon,
[4-((3-Fluor-5-trifluormethyl-pyridin-2-yl)-piperazin-1-yl]-[5-methansulfonyl-2-((S)-2,2,2-trifluor-1-methyl-ethoxy)-phenyl]-methanon oder
[4-((3-Fluor-5-trifluormethyl-pyridin-2-yl)-piperazin-1-yl]-[5-methansulfonyl-2-(2,2,2-trifluor-1,1-dimethyl-ethoxy)-phenyl]-methanon.

3. Eine pharmazeutische Kombination gemäß Anspruch 2, umfassend einen atypischen antipsychotischen Arzneistoff, ausgewählt aus der Gruppe bestehend aus Risperidon, Paliperidon, Olanzapin, Aripiprazol, Quetiapin oder Ziprasidon, und den GlyT1-Rezeptor-Antagonisten [4-(3-Fluor-5-trifluormethyl-pyridin-2-yl)-piperazin-1-yl]-[5-methansulfonyl-2-(2,2,2-trifluor-1-methyl-ethoxy)-phenyl]-methanon.

4. Eine pharmazeutische Kombination, umfassend einen atypischen antipsychotischen Arzneistoff, ausgewählt aus der Gruppe bestehend aus Olanzapin oder Risperidon, und einen GlyT1-Rezeptor-Antagonisten, bei dem es sich um 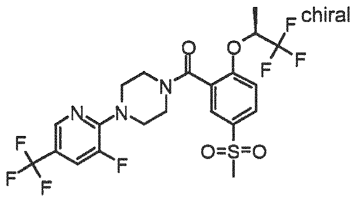 handelt, und pharmazeutisch verträgliche Säureadditionssalze davon sowie enantiomere Formen davon.

5. Eine pharmazeutische Kombination gemäß Anspruch 1, umfassend einen atypischen antipsychotischen Arzneistoff und einen GlyT1-Rezeptor-Antagonisten, zur Verwendung bei der Behandlung von positiven und negativen Symptomen bei Schizophrenie.

6. Die Verwendung einer pharmazeutischen Kombination, umfassend einen atypischen antipsychotischen Arzneistoff und einen GlyT1-Rezeptor-Antagonisten der Formel 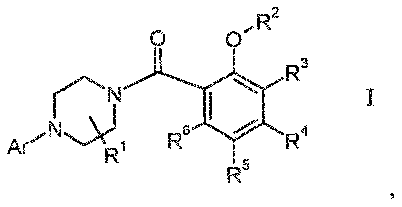 wobei
Ar ein substituierter 6-gliedriger Heteroarylrest ist, der ein, zwei oder drei Stickstoffatome enthält, und wobei die Heteroarylreste mit einem oder mehreren Substituenten substituiert sein können, ausgewählt aus der Gruppe bestehend aus Halogen, (C₁-C₆)-Alkyl oder (C₁-C₆)-Alkyl, substituiert mit Halogen;
R¹ Wasserstoff oder (C₁-C₆)-Alkyl ist;
R² (C₁-C₆)-Alkyl, substituiert mit Halogen, ist,
R³, R⁴ und R⁶ unabhängig voneinander Wasserstoff, Halogen, (C₁-C₆)-Alkyl oder (C₁-C₆)-Alkoxy sind;
R⁵ SO₂R¹⁰ ist;
R¹⁰ (C₁-C₆)-Alkyl, gegebenenfalls substituiert mit Halogen, ist,
und pharmazeutisch verträglicher Säureadditionssalze davon sowie enantiomere Formen davon zur Herstellung eines Medikaments zur Behandlung von positiven und negativen Symptomen bei Schizophrenie.

7. Die Verwendung einer pharmazeutischen Kombination gemäß Anspruch 6, umfassend einen atypischen antipsychotischen Arzneistoff, ausgewählt aus der Gruppe bestehend aus Risperidon, Paliperidon, Olanzapin, Aripiprazol, Quetiapin oder Ziprasidon, und einen GlyT1-Rezeptor-Antagonisten, ausgewählt aus
rac-[4-(3-Chlor-5-trifluormethyl-pyridin-2-yl)-piperazin-1-yl]-[5-methansulfonyl-2-(2,2,2-trifluor-1-methyl-ethoxy)-phenyl]-methanon,
rac-[5-Methansulfonyl-2-(2,2,2-trifluor-1-methyl-ethoxy)-phenyl]-[4-(5-trifluormethyl-pyridin-2-yl)-piperazin-1-yl]-methanon,
rac-[4-(5-Brom-pyridin-2-yl)-piperazin-1-yl]-[5-methansulfonyl-2-(2,2,2-trifluor-1-methyl-ethoxy)-phenyl]-methanon,
rac-[4-(3-Fluor-5-trifluormethyl-pyridin-2-yl)-piperazin-1-yl]-[5-methansulfonyl-2-(2,2,2-trifluor-1-methyl-ethoxy)-phenyl]-methanon,
rac-[5-Methansulfonyl-2-(2,2,2-trifluor-1-methyl-ethoxy)-phenyl]-[4-(6-trifluormethyl-pyridin-2-yl)-piperazin-1-yl]-methanon,
[5-Methansulfonyl-2-((S oder R)-2,2,2-trifluor-1-methyl-ethoxy)-phenyl]-[4-(5-trifluormethyl-pyridin-2-yl)-piperazin-1-yl]-methanon,
[5-Methansulfonyl-2-((R oder S)-2,2,2-trifluor-1-methyl-ethoxy)-phenyl]-[4-(5-trifluormethyl-pyridin-2-yl)-piperazin-1-yl]-methanon,
[4-((3-Fluor-5-trifluormethyl-pyridin-2-yl)-piperazin-1-yl]-[5-methansulfonyl-2-((S)-2,2,2-trifluor-1-methyl-ethoxy)-phenyl]-methanon oder
[4-((3-Fluor-5-trifluormethyl-pyridin-2-yl)-piperazin-1-yl]-[5-methansulfonyl-2-(2,2,2-trifluor-1,1-dimethyl-ethoxy)-phenyl]-methanon
zur Herstellung eines Medikaments zur Behandlung von positiven und negativen Symptomen bei Schizophrenie.

8. Die Verwendung einer pharmazeutischen Kombination gemäß Anspruch 7, umfassend einen atypischen antipsychotischen Arzneistoff, ausgewählt aus der Gruppe bestehend aus Risperidon, Paliperidon, Olanzapin, Aripiprazol, Quetiapin oder Ziprasidon, und den GlyT1 -Rezeptor-Antagonisten [4-(3-Fluor-5-trifluormethyl-pyridin-2-yl)-piperazin-1 - yl]-[5-methansulfonyl-2-(2,2,2-trifluor-1-methyl-ethoxy)-phenyl]-methanon zur Herstellung eines Medikaments zur Behandlung von positiven und negativen Symptomen bei Schizophrenie.

9. Die Verwendung pharmazeutischen Kombination gemäß Anspruch 8, umfassend einen atypischen tipsychotischen Arzneistoff, ausgewählt aus der Gruppe bestehend aus Olanzapin oder Risperidon, und einen GlyT1-Rezeptor-Antagonisten, bei dem es sich um 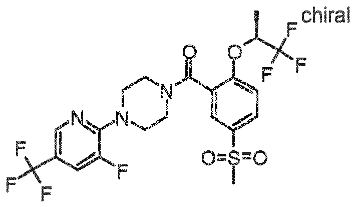 handelt, und pharmazeutisch verträglicher Säureadditionssalze davon, sowie enantiomerer Formen davon.

## Revendications

1. Combinaison pharmaceutique comprenant un médicament antipsychotique atypique et un antagoniste de récepteur GlyT1 de formule 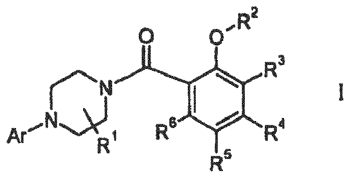 où
Ar est un groupe hétéroaryle à 6 chaînons substitué, contenant un, deux ou trois atomes d'azote, et où les groupes hétéroaryle peuvent être substitués par un ou plusieurs substituants choisis dans le groupe consistant en halogène, (C₁-C₆)-alkyle et (C₁-C₆)-alkyle substitué par halogène;
R¹ est hydrogène ou (C₁-C₆)-alkyle;
R² est (C₁-C₆)-alkyle substitués par halogène,
R³, R⁴ et R⁶ sont indépendamment les uns des autres hydrogène, halogène, (C₁-C₆)-alkyle ou (C₁-C₆)-alcoxy;
R⁵ est SO₂R¹⁰;
R¹⁰ est (C₁-C₆)-alkyle éventuellement substitué par halogène,
et ses sels d'addition d'acide pharmaceutiquement acceptables, ainsi que ses formes énantiomères.

2. Combinaison pharmaceutique selon la revendication 2 comprenant un médicament antipsychotique atypique, choisi dans le groupe consistant en rispéridone, palipéridone, olanzapine, aripiprazole, quetiapine et ziprasidone et un antagoniste de récepteur GlyT1 choisi parmi
la rac-[4-(3-chloro-5-trifluorométhyl-pyridin-2-yl)-pipérazin-1-yl]-[5-méthane-sulfonyl-2-(2,2,2-trifluoro-1-méthyl-éthoxy)-phényl]-méthanone,
la rac-[5-méthanesulfonyl-2-(2,2,2-trifluoro-1-méthyl-éthoxy)-phényl]-[4-(5-trifluorométhyl-pyridin-2-yl)-pipérazin-1-yl]-méthanone,
la rac-[4-(5-bromo-pyridin-2-yl)-pipérazin-1-yl]-[5-méthanesulfonyl-2-(2,2,2-trifluoro-1--méthyléthoxy)-phényl]-méthanone,
la rac-[4-(3--fluoro-5-trifluorométhyl-pyridin-2-yl)-pipérazin-1-yl]-[5-méthane-sulfonyl-2-(2,2,2-trifluoro-1-méthyl-éthoxy)-phényl]-méthanone,
la rac-[5-méthanesulfonyl-2-(2,2,2-trifluoro-1-méthyl-éthoxy)-phényl]-[4-(6-trifluorométhyl-pyridin-2-yl)-pipérazin-1-yl]-méthanone,
la [5-méthanesulfonyl-2-((S ou R)-2,2,2-trifluoro-1-méthyl-éthoxy)-phényl]-[4-(5-trifluorométhyl-pyridin-2-yl)-pipérazin-1-yl]-méthanone,
la [5-méthanesulfonyl-2-((R ou S)-2,2,2-trifluoro-1-méthyl-éthoxy)-phényl]-[4-(5-trifluorométhyl-pyridin-2-yl)-pipérazin-1-yl]-méthanone,
la [4-(3-fluoro-5-trifluorométhyl-pyridin-2-yl)-pipérazin-1-yl]-[5-méthanesulfonyl-2-((S)-2,2,2 trifluoro-1-méthyl-éthoxy)-phényl]-méthanone et
la [4-(3-fluoro-5-trifluorométhyl-pyridin-2-yl)-pipérazin-1-yl]-[5-méthanesulfonyl-2-(2,2,2-trifluoro-1,1-diméthyl-éthoxy)-phényl]-méthanone.

3. Combinaison pharmaceutique selon la revendication 2 comprenant un médicament antipsychotique atypique, choisi dans le groupe consistant en rispéridone, palipéridone, olanzapine, aripiprazole, quetiapine et ziprasidone et l'antagoniste de récepteur GlyT1 [4-(3-fluoro-5-trifluorométhyl-pyridin-2-yl)-pipérazin-1-yl]-[5-méthanesulfonyl-2-(2,2,2-trifluoro-1-méthyl-éthoxy)-phényl]-méthanone.

4. Combinaison pharmaceutique comprenant un médicament antipsychotique atypique choisi dans le groupe consistant en olanzapine et rispéridone et un antagoniste de récepteur GlyT1 qui est 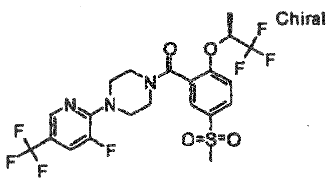 et ses sels d'addition d'acide pharmaceutiquement acceptables, ainsi que ses formes énantiomères.

5. Combinaison pharmaceutique selon la revendication 1 comprenant un médicament antipsychotique atypique et un antagoniste de récepteur GlyT1 destinée à être utilisée dans le traitement des symptômes positifs et négatifs dans la schizophrénie.

6. Utilisation d'une combinaison pharmaceutique comprenant un médicament antipsychotique atypique et un antagoniste de récepteur GlyT1 de formule 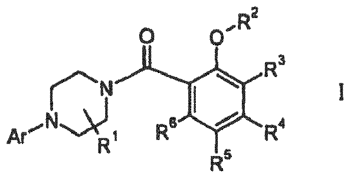 où
Ar est un groupe hétéroaryle à 6 chaînons substitué, contenant un, deux ou trois atomes d'azote, et où les groupes hétéroaryle peuvent être substitués par un ou plusieurs substituants choisis dans le groupe consistant en halogène, (C₁-C₆)-alkyle et (C₁-C₆)-alkyle substitué par halogène;
R¹ est hydrogène ou (C₁-C₆)-alkyle;
R² est (C₁-C₆)-alkyle substitués par halogène,
R³, R⁴ et R⁶ sont indépendamment les uns des autres hydrogène, halogène, (C₁-C₆)-alkyle ou (C₁-C₆)-alcoxy;
R⁵ est SO₂R¹⁰;
R¹⁰ est (C₁-C₆)-alkyle éventuellement substitué par halogène,
et ses sels d'addition d'acide pharmaceutiquement acceptables, ainsi que ses formes énantiomères pour la préparation d'un médicament pour le traitement des symptômes positifs et négatifs dans la schizophrénie.

7. Utilisation d'une combinaison pharmaceutique selon la revendication 6 comprenant un médicament antipsychotique atypique, choisi dans le groupe consistant en rispéridone, palipéridone, olanzapine, aripiprazole, quetiapine et ziprasidone et un antagoniste de récepteur GlyT1 choisi parmi
la rac-[4-(3-chloro-5-trifluorométhyl-pyridin-2-yl)-pipérazin-1-yl]-[5-méthane-sulfonyl-2-(2,2,2-trifluoro-1-méthyl-éthoxy)-phényl]-méthanone,
la rac-[5-méthanesulfonyl-2-(2,2,2-trifluoro-1-méthyl-éthoxy)-phényl]-[4-(5-trifluorométhyl-pyridin-2-yl)-pipérazin-1-yl]-méthanone,
la rac-[4-(5-bromo-pyridin-2-yl)-pipérazin-1-yl]-[5-méthanesulfonyl-2-(2,2,2-trifluoro-1-méthyléthoxy)-phényl]-méthanone,
la rac-[4-(3-fluoro-5-trifluorométhyl-pyridin-2-yl)-pipérazin-1-yl]-[5-méthanesulfonyl-2-(2,2,2-trifluoro-1-méthyl-éthoxy)-phényl]-méthanone,
la rac-[5-méthanesulfonyl-2-(2,2,2-trifluoro-1-méthyl-éthoxy)-phényl]-[4-(6-trifluorométhyl-pyridin-2-yl)-pipérazin-1-yl]-méthanone,
la [5-méthanesulfonyl-2-((S ou R)-2,2,2-trifluoro-1-méthyl-éthoxy)-phényl]-[4-(5-trifluorométhyl-pyridin-2-yl)-pipérazin-1-yl]-méthanone,
la [5-méthanesulfonyl-2-((R ou S)-2,2,2-trifluoro-1-méthyl-éthoxy)-phényl]-[4-(5-trifluorométhyl-pyridin-2-yl)-pipérazin-1-yl]-méthanone,
la [4-(3-fluoro-5-trifluorométhyl-pyridin-2-yl)-pipérazin-1-yl]-[5-méthanesulfonyl-2-((S)-2,2,2 trifluoro-1-méthyl-éthoxy)-phényl]-méthanone et
la [4-(3-fluoro-5-trifluorométhyl-pyridin-2-yl)-pipérazin-1-yl]-[5-méthanesulfonyl-2-(2,2,2-trifluoro-1,1-diméthyl-éthoxy)-phényl]-méthanone pour la préparation d'un médicament pour le traitement des symptômes positifs et négatifs dans la schizophrénie.

8. Utilisation d'une combinaison pharmaceutique selon la revendication 7 comprenant un médicament antipsychotique atypique, choisi dans le groupe consistant en rispéridone, palipéridone, olanzapine, aripiprazole, quetiapine et ziprasidone et l'antagoniste de récepteur GlyT1 [4-(3-fluoro-5-trifluorométhyl-pyridin-2-yl)-pipérazin-1-yl]-[5-méthanesulfonyl-2-(2,2,2-trifluoro-1-méthyl-éthoxy)-phényl]-méthanone pour la préparation d'un médicament pour le traitement des symptômes positifs et négatifs dans la schizophrénie.

9. Utilisation d'une combinaison pharmaceutique selon la revendication 8, comprenant un médicament antipsychotique atypique choisi dans le groupe consistant en olanzapine et rispéridone et un antagoniste de récepteur GlyT1 qui est 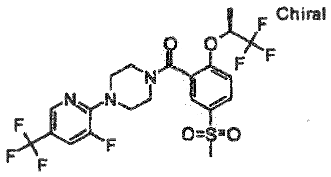 et ses sels d'addition d'acide pharmaceutiquement acceptables, ainsi que ses formes énantiomères.
